# EUROPEAN PATENT APPLICATION

(11) **EP 4 321 532 A1**
(43) Date of publication of application: **14.02.2024**
(21) Application number: 22784326.5
(22) Date of filing: 09.02.2022
(51) Int. Cl.: C07K 16/18, C07K 17/00, G01N 33/50, G01N 33/68, G01N 33/96, G01N 27/62, C07K 14/47

(54) **METHOD FOR CREATING CALIBRATION CURVE AND METHOD FOR MEASURING AMYLOID BETA-RELATED PEPTIDE**

(30) Priority: 05.04.2021 JP 2021063916
(71) Applicant: Shimadzu Corporation, Nakagyo-ku, Kyoto-shi, Kyoto 604-8511 (JP)
(72) Inventor: KANEKO, Naoki, Kyoto-shi, Kyoto 604-8511 (JP)
(74) Representative: Müller-Boré & Partner Patentanwälte PartG mbB
(86) International application number: PCT/JP2022/005016
(87) International publication number: WO 2022/215341

(57) **Abstract**

Included is the steps of providing a plurality of standard solutions containing amyloid β-related peptide and a surfactant and having different concentrations of the amyloid β-related peptide; mixing a solution containing internal standard peptide with each of the plurality of standard solutions; performing immunoprecipitation and mass spectrometry on a plurality of calibration curve solutions; standardizing signal intensity (A) in the amyloid β-related peptide with the signal intensity (B) in the internal standard peptide for each of the plurality of calibration curve solutions; and calculating a regression equation of a calibration curve based on a plurality of normalized intensities and the concentration of the amyloid β-related peptide.

## Description

### TECHNICAL FIELD

The present invention relates to a method for creating a calibration curve and a method for measuring amyloid β-related peptide.

### BACKGROUND ART

Alzheimer's disease is a leading cause of dementia, its afflicted people increase more and more in recent years, and its research has become even more important. In the development of Alzheimer's disease, Aβ-related peptides such as amyloid β (AB) generated by cleavage of amyloid precursor protein (APP) are deeply involved. Then, it has been reported that a plurality of Aβ-related peptides in blood are detected by combining immunoprecipitation and mass spectrometry, and a specific Aβ-related peptide ratio detected is promising as a blood biomarker of amyloid accumulation in brain (see Non-Patent Documents 1 to 2 and Patent Documents 1 to 2).

### PRIOR ART DOCUMENTS

### PATENT DOCUMENTS

Patent Document 1: Japanese Patent Application Laid-Open No. 2017-20980
Patent Document 2: WO2015/178398

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K.: Novel plasma biomarker surrogating cerebral amyloid deposition. Proc Jpn Acad Ser B Phys Biol Sci. 2014;90(9):353-364.

Non-Patent Document 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K.: High performance plasma amyloid-β biomarkers for Alzheimer's disease. Nature. 2018;554(7691):249-254.

### SUMMARY OF THE INVENTION

### PROBLEMS TO BE SOLVED BY THE INVENTION

In the measurement methods described in these documents, the Aβ-related peptide ratio, that is, a relative amount of each Aβ-related peptide is calculated from signal intensity obtained by mass spectrometry.

Meanwhile, knowing not only the Aβ-related peptide ratio but also a presence concentration of each Aβ-related peptide in a measurement sample is important information for grasping how the Aβ-related peptide behaves in conjunction with progress of a disease state.

In order to measure the concentration of the Aβ-related peptide, a method using a calibration curve is studied. Specifically, internal standard peptide whose concentration is known is mixed in a measurement sample whose concentration is to be measured, and mass spectrometry is performed to calculate a signal intensity ratio of the peptide to be measured to the internal standard peptide. Subsequently, the signal intensity ratio is applied to a calibration curve on which a relationship between the concentration of the peptide to be measured and the signal intensity has been investigated in advance to determine the concentration. Non-Patent Document 2 describes a calibration curve of Aβ-related peptides (AB1-42, Aβ1-40, APP669-711). This calibration curve is created by detecting the signal intensity of each peptide by mass spectrometry using a calibration curve solution obtained by adding the Aβ-related peptide to BSA-containing PBS (bovine serum albumin-containing phosphate buffered saline).

Since accuracy of the calibration curve reflects accuracy of the concentration of the Aβ-related peptide, it is important to improve the accuracy of the calibration curve.

An object of the present invention is to provide a method for accurately creating a calibration curve for quantifying amyloid β-related peptide and a method for accurately quantifying the amyloid β-related peptide.

### MEANS FOR SOLVING THE PROBLEMS

A first aspect of the present invention is a method for creating a calibration curve for quantifying amyloid β-related peptide. The method for creating a calibration curve includes the steps of providing a plurality of standard solutions containing the amyloid β-related peptide and a surfactant and having different concentrations of the amyloid β-related peptide; mixing a solution containing internal standard peptide with each of the plurality of standard solutions to obtain a plurality of calibration curve solutions; performing immunoprecipitation and mass spectrometry on the plurality of calibration curve solutions to obtain signal intensity (A) in the amyloid β-related peptide and a signal intensity (B) in the internal standard peptide for each of the plurality of calibration curve solutions; standardizing the signal intensity (A) with the signal intensity (B) for each of the plurality of calibration curve solutions to obtain a plurality of normalized intensities; and calculating a regression equation of a calibration curve based on the plurality of normalized intensities and the concentration of the amyloid β-related peptide corresponding to the plurality of normalized intensities.

A second aspect of the present invention is a method for measuring amyloid β-related peptide. In this measurement method, the amyloid β-related peptide in a measurement sample is quantified using the calibration curve obtained by the creation method of the first aspect.

### EFFECTS OF THE INVENTION

According to the first aspect of the present invention, an accurate calibration curve for quantifying amyloid β-related peptide can be obtained. According to the second aspect of the present invention, amyloid β-related peptide can be accurately quantified.

### BRIEF DESCRIPTION OF THE DRAWINGS

FIG. 1 is a calibration curve (linear regression line, no weighting) of A61-40 created in Example, in which the vertical axis represents normalized intensity and the horizontal axis represents peptide concentration.
FIG. 2 is a calibration curve (quadratic curve) of Aβ1-40 created in Example.
FIG. 3 is a calibration curve (linear regression line, no weighting) of A61-42 created in Example.
FIG. 4 is a calibration curve (quadratic curve) of Aβ1-42 created in Example.
FIG. 5 is a calibration curve (linear regression line, no weighting) of APP669-711 created in Example.
FIG. 6 is a calibration curve (quadratic curve) of APP669-711 created in Example.
FIG. 7 is a graph showing adsorption loss of peptide to a tube, and shows signal values of standardized SIL-Aβ1-38 when each peptide solution is transferred and when each peptide solution is not transferred.

### MODE FOR CARRYING OUT THE INVENTION

### 1. First aspect (method for creating calibration curve)

A first aspect is a method for creating (producing) a calibration curve for quantifying amyloid β-related peptide (hereinafter, abbreviated as "Aβ-related peptide"). A first aspect includes a standard solution provision step, a calibration curve solution preparation step, an immunoprecipitation-mass spectrometry step, a signal standardization step, and a calculation step in this order. Each step will be described below.

### [Standard solution provision step]

In this step, a plurality of standard solutions are provided.

The standard solution is a standard solution for an object to be measured containing Aβ-related peptide and a surfactant. In the standard solution, the concentration of the Aβ-related peptide is known. As the standard solution, a plurality of solutions having different concentrations of Aβ-related peptides are provided. Specifically, 5 or more and 20 or less kinds of standard solutions having an Aβ-related peptide concentration in a range of 1 pM to 500 pM are provided.

The "amyloid β-related peptide (AB-related peptide)" is peptide including Aβ generated by cleavage of amyloid precursor protein (APP) and even a part of the sequence of Aβ. Specifically, this is disclosed in WO 2015/178398 and the like. As the Aβ-related peptide, a peptide to be measured may be arbitrarily selected from the peptides, from the viewpoint of being suitable as a blood biomarker of amyloid accumulation in brain, at least one of Aβ1-40 (SEQ ID NO: 1, see Table 1), Aβ1-42 (SEQ ID NO: 2), and APP669-711 (SEQ ID NO: 3) is preferable, and a combination of these three is more preferable.

**[Table 1]**

| No. | Name | Sequence |
|---|---|---|
| 1 | A*β*1 - 40 | DAEFRHDSGYEVHHQKLVFFAEDVGSNKGAIIGLVVGGVV |
| 2 | A*β*1 - 42 | DAEFRHDSGYEVHHQKLVFFAEDVGSNKGAIIGLMVGGVVIA |
| 3 | APP669 - 711 | VKMDAEFRHDSGYEVHHQKLVFFAEDVGSNKGAIIGLMVGGVV |

Examples of the surfactant include nonionic surfactants, amphoteric surfactants, anionic surfactants, and cationic surfactants. Preferred examples include nonionic surfactants. By using the nonionic surfactant, it is possible to suppress easy ionization and detection of the surfactant when mass spectrometry is performed in a state where the surfactant remains, and to reduce an influence on creation of a calibration curve.

Examples of the nonionic surfactant include a surfactant having maltose in a hydrophilic portion, a surfactant having trehalose in a hydrophilic portion, a surfactant having glucose in a hydrophilic portion; and a surfactant having a polyoxyethylene group in a hydrophilic portion.

Examples of the surfactant having maltose include n-nonyl-β-D-maltoside (NM), n-nonyl-β-D-thiomaltoside (NTM), n-decyl-β-D-maltoside (DM), n-undecyl-β-D-maltoside (UDM), and n-dodecyl-β-D-maltoside (DDM).

Examples of the surfactant having trehalose include α-D-glucopyranosyl α-D-glucopyranoside monooctanoate (trehalose C8), α-D-glucopyranosyl α-D-glucopyranoside monododecanoate (trehalose C12), and α-D-glucopyranosyl α-D-glucopyranoside monomyristate (trehalose C14).

Examples of the surfactant having glucose include n-decyl-β-D-glucoside (DG) and n-octyl-β-D-glucoside (OG).

Examples of the surfactant having a polyoxyethylene group include polyoxyethylene sorbitan monolaurate (Tween (registered trademark) 20), polyoxyethylene sorbitan monooleate (Tween (registered trademark) 80), and polyoxyethylene p-t-octylphenol (Triton (registered trademark) X-100).

Preferred examples include a surfactant having maltose and a surfactant having a polyoxyethylene group. A surfactant having a polyoxyethylene group is more preferable from the viewpoint of further suppressing the adhesion of the Aβ-related peptide to a container and measuring a more accurate calibration curve. On the other hand, from the viewpoint that handling is facilitated, the surfactant can be relatively easily removed, and an influence of the remaining surfactant on the mass spectrometry can be reduced, the same type of surfactant as the surfactant used in immunoprecipitation (for example, a surfactant having maltose) is more preferable.

These surfactants can be used singly or in combination of two or more types thereof.

The concentration of the surfactant is, for example, 0.01% (w/v) or more, preferably 0.05% (w/v) or more, and is, for example, 10% (w/v) or less, preferably 3% (w/v) or less.

A solvent used in the standard solution is preferably a buffer. Examples of the buffer include a Tris buffer, a phosphate buffer, a HEPES buffer, and an ammonium acetate buffer. The buffer of the standard solution is preferably a neutral buffer, and the pH thereof is, for example, 6.0 or more, preferably 6.5 or more, and is, for example, 8.5 or less, preferably 8.0 or less.

The standard solution may contain, for example, an additive such as BSA (bovine serum albumin).

The standard solution having a plurality of concentrations can be prepared, for example, by mixing a predetermined amount of commercially available Aβ-related peptide with a predetermined amount of aqueous ammonia, a surfactant containing buffer, and the like while calculating each mixing amount.

### [Calibration curve solution preparation step]

In this step, an internal standard-containing solution is mixed with each of a plurality of standard solutions. As a result, a plurality of calibration curve solutions are obtained.

The internal standard-containing solution is an internal standard solution containing internal standard peptide. In the internal standard-containing solution, the concentration of the internal standard peptide is known.

The internal standard peptide is a peptide different from the peptide to be measured, and examples thereof include stable isotope-labeled Aβ-related peptides (SIL-Aβ-related peptides).

The internal standard-containing solution preferably contains a surfactant. As a result, it is possible to correctly detect the signal intensity of the internal standard peptide by suppressing adhesion of the internal standard peptide to the container and thus adhesion loss, and to create a more accurate calibration curve. Examples of the surfactant used in the internal standard-containing solution include similar surfactants to those used in the standard solution. The surfactant concentration in the internal standard-containing solution is similar to that in the standard solution.

Examples of the solvent used for the internal standard-containing solution include similar solvents to those used for the standard solution. The internal standard-containing solution can be prepared, for example, by mixing a surfactant containing buffer with a predetermined amount of the internal standard peptide.

The internal standard-containing solution is appropriately mixed with each of the plurality of standard solutions. Specifically, each standard solution is added to the internal standard-containing solution. A mixing ratio (volume ratio) between each standard solution and the internal standard-containing solution may be, for example, about 10: 1 to 1: 10, and mixing is preferably performed in equal amounts.

As a result, a plurality of calibration curve solutions having different Aβ-related peptide concentrations are obtained. For example, 5 or more and 20 or less kinds of calibration curve solutions having an AB-related peptide concentration in the range of 1 pM to 500 pM are prepared. The concentration of the internal standard peptide among the plurality of calibration curve solutions is preferably the same.

### [Immunoprecipitation-mass spectrometry step]

In this step, immunoprecipitation and mass spectrometry are sequentially performed on each of the plurality of calibration curve solutions. Specifically, a first bonding step, a first washing step, a first elution step, a neutralization step, a second bonding step, a second washing step, a second elution step, and a detection step are sequentially performed.

### (First bonding step)

In this step, the calibration curve solution is brought into contact with a first carrier. For example, the calibration curve solution is mixed with the first carrier. As a result, a first conjugate in which the Aβ-related peptide is bonded to the first carrier is obtained.

The first carrier may be any carrier to which the Aβ-related peptide can be bonded, and examples thereof include an antibody-immobilizing carrier.

The antibody immobilized on the first carrier is an antibody (AB-related peptide antibody) having an antigen binding site capable of recognizing Aβ-related peptide, and examples thereof include an immunoglobulin having an antigen binding site capable of recognizing Aβ-related peptide or a fragment thereof.

Examples of the immunoglobulin include IgG (IgG1, IgG2, IgG3, IgG4), IgM, IgA, IgY, IgD, and IgE. The immunoglobulin is appropriately determined according to a substance to be analyzed, and a known immunoglobulin may be adopted. Examples of the immunoglobulin fragment include F(ab')2, F(ab'), F(ab), Fd, Fv, L chain, and H chain. When the substance to be analyzed is Aβ-related peptide, the immunoglobulin or a fragment thereof (anti-AB-related peptide antibody) having an antigen binding site capable of recognizing the AB-related peptide is, for example, 6E 10, 4G8, 1E11, 11A50-B10, 12F4, 9C4, 82E1, 12B2, 1A10, a fragment thereof, or the like. The antibody may be either a monoclonal antibody or a polyclonal antibody.

Examples of the material of the first carrier include agarose, sepharose, dextran, silica gel, polyacrylamide, polystyrene, polyethylene, polypropylene, polyester, polyacrylonitrile, a (meth)acrylic acid-based polymer, a fluororesin, a metal complex resin, glass, metal, and a magnetic body.

The shape of the first carrier may be any shape such as a spherical shape (including a bead shape), a plate shape, a needle shape, or an irregular shape, and may be a flow path wall in a microdevice or the like.

Before the first bonding step, pretreatment for removing antibodies such as IgG and IgM may be performed as necessary.

### (First washing step)

In this step, after the first bonding step, the first conjugate is washed using a washing liquid.

As a washing method, a known method may be employed, and washing is preferably performed using a washing liquid containing a surfactant. For example, first, washing is performed using a neutral buffer containing a surfactant as a washing liquid, and subsequently, washing is performed using a neutral buffer containing no surfactant as a washing liquid.

As the neutral buffer containing a surfactant, those similar to the surfactant and neutral buffer exemplified in the standard solution can be used. The surfactant concentration in the washing liquid is similar to that in the standard solution. As a result, for example, unnecessary components (blood proteins, lipids, glycolipids, etc.) having high hydrophobicity can be effectively removed.

As the neutral buffer containing no surfactant, a neutral buffer similar to the neutral buffer exemplified in the standard solution can be used. This makes it possible to suppress foaming caused by the surfactant remaining in the first conjugate.

As the washing method, a general method may be employed, and examples thereof include a method of stirring a carrier in a washing liquid and a method of spraying a washing liquid from a washing nozzle.

After washing with these neutral buffers, washing with water may be further performed as necessary.

### (First elution step)

In this step, after the first washing step, the first conjugate is brought into contact with a first acidic solution. As a result, the Aβ-related peptide is dissociated from the first conjugate, and the Aβ-related peptide is eluted into the first acidic solution. As a result, a first eluate containing the Aβ-related peptide is obtained.

Examples of the first acidic solution include an acidic aqueous solution such as a glycine buffer and hydrochloric acid, and preferably include a glycine buffer. The pH of the first acidic solution is, for example, 3.5 or less, preferably 3.0 or less, and is, for example, 0.5 or more, preferably 1.0 or more.

The first acidic solution preferably contains a surfactant. This makes it possible to more reliably dissociate the Aβ-related peptide from the first conjugate. Adhesion of the eluted Aβ-related peptide to a container such as a tube or a microplate is suppressed. Therefore, a recovery rate of the Aβ-related peptide can be reliably improved.

Examples of the surfactant used in the first acidic solution include similar surfactants to those exemplified in the standard solution. The surfactant concentration in the first acidic solution is similar to that in the standard solution.

### (Neutralization step)

In this step, after the first elution step, the first eluate is mixed with a neutral buffer. As a result, the first eluate is neutralized to obtain a purification solution containing Aβ-related peptide.

The neutral buffer used in the neutralization step may contain a surfactant. In particular, when the first acidic solution contains no surfactant, it is preferable that the neutral buffer contains a surfactant. This makes it possible to suppress non-specific adsorption to a second conjugate in the second bonding step.

Examples of the neutral buffer and the surfactant used in the neutralization step include those similar to the neutral buffer and the surfactant exemplified in the standard solution. The surfactant concentration in the neutral buffer is similar to that in the standard solution.

The pH of the purification solution is neutral, and is, for example, 6.0 or more, preferably 6.5 or more, and is, for example, 8.5 or less, preferably 8.0 or less. Accordingly, in the second bonding step, the bonding efficiency can be improved.

### (Second bonding step)

In this step, after the neutralization step, the purification solution is brought into contact with a second carrier. As a result, a second conjugate in which the Aβ-related peptide is bonded to the second carrier is obtained.

The second carrier is preferably an antibody-immobilizing carrier, and specific examples thereof include those similar to the antibody-immobilizing carrier exemplified for the first carrier.

### (Second washing step)

In this step, after the second bonding step, the second conjugate is washed using a washing liquid.

As the washing method, a known method may be adopted, and a method similar to the washing method exemplified in the first washing step may be performed. For example, first, washing is performed using a neutral buffer containing a surfactant as a washing liquid, and subsequently, washing is performed using a neutral buffer containing no surfactant as a washing liquid.

### (Second elution step)

In this step, after the second washing step, the second conjugate is brought into contact with a second acidic solution. As a result, the Aβ-related peptide is dissociated from the second conjugate, and the Aβ-related peptide is eluted into the second acidic solution. As a result, a second eluate containing the Aβ-related peptide is obtained.

Examples of the second acidic solution include those similar to the first acidic solution exemplified in the first elution step, and preferably include hydrochloric acid.

The second acidic solution preferably contains a volatile organic solvent. As a result, the Aβ-related peptide can be efficiently dissociated from the second conjugate and can be eluted in the second acidic solution, and the recovery rate of the Aβ-related peptide can be improved.

Examples of the volatile organic solvent include organic solvents miscible with water in an arbitrary ratio, and examples thereof include acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate, and preferably include acetonitrile, methanol, ethanol, acetone, and isopropanol. These solvents can be used singly or in combination of two or more kinds thereof.

The concentration of the volatile organic solvent in the second acidic solution is, for example, 10% (v/v) or more, preferably 25% (v/v) or more, and is, for example, 90% (v/v) or less, preferably 80% (v/v) or less. When the concentration is within the above range, the Aβ-related peptide can be efficiently dissociated from the second carrier, and sensitivity (S/N ratio) at the time of mass spectrometry can be improved.

The second acidic solution preferably further contains an amino acid such as methionine. As a result, oxidation of the Aβ-related peptide can be reduced and analysis precision can be improved until placement in a mass spectrometer and start of analysis. The concentration of amino acid in the second acidic solution is, for example, 0.01 mM or more, preferably 0.05 mM or more, and is, for example, 5 mM or less, preferably 1 mM or less.

### (Analysis step)

In this step, after the second elution step, the Aβ-related peptide in the second eluate is detected by mass spectrometry.

Examples of the mass spectrometry include MALDI (Matrix Assisted Laser Desorption/Ionization), ESI (Electrospray ionization), and APCI (Atmospheric Pressure Chemical Ionization). MALDI is preferable from the viewpoint that the measurement can be performed without using liquid chromatography, so that loss such as adsorption can be reduced, and the Aβ-related peptide can be reliably ionized even if some contaminants coexist.

For detection of MALDI, for example, MALDI-TOF (Matrix-Assisted Laser Desorption / Ionization-Time of Flight) type mass spectrometer, MALDI-IT (Matrix-Assisted Laser Desorption / Ionization-Ion Trap) type mass spectrometer, MALDI-IT-TOF (Matrix-Assisted Laser Desorption / Ionization-Ion Trap-Time of Flight) type mass spectrometer, and MALDI-FTICR (Matrix-Assisted Laser Desorption / Ionization-Fourier Transform Ion Cyclotron Resonance) type mass spectrometer, or the like may be operated according to a conventional method.

Upon detection with MALDI, a matrix is placed, for example, by dropping a matrix-containing solution onto a MALDI plate and drying it.

Examples of the matrix include α-cyano-4-hydroxycinnamic acid (CHCA), 2,5-dihydroxybenzoic acid, sinapinic acid, and 3-aminoquinoline. These matrices can be used singly or in combination of two or more kinds thereof.

Examples of the solvent contained in the matrix include acetonitrile, trifluoroacetic acid, methanol, ethanol, and water. These matrices can be used singly or in combination of two or more kinds thereof.

The matrix concentration in a matrix-containing solvent is, for example, 0.1 mg/mL or more, preferably 0.5 mg/mL or more, and is, for example, 50 mg/mL or less, preferably 10 mg/mL or less.

Preferably, a matrix additive is used in combination with the matrix. Examples of the matrix additive include a phosphonic acid group-containing compound and an ammonium salt, and preferably include a phosphonic acid group-containing compound from the viewpoint of being able to suppress an adverse effect on the background due to remaining of the washing solution. Examples of the phosphonic acid group-containing compound include phosphonic acid, methylphosphonic acid, phenylphosphonic acid, 1-naphthylmethylphosphonic acid, methylenediphosphonic acid (MDPNA), ethylene diphosphonic acid, ethane-1-hydroxy-1,1-diphosphonic acid, nitrilotriphosphonic acid, and ethylene diaminotetraphosphonic acid.

The matrix additive concentration in the matrix-containing solvent is, for example, 0.01% (w/v) or more, preferably 0.1% (w/v) or more, and is, for example, 10% (w/v) or less, preferably 1% (w/v) or less.

As a result, in each second eluate (thus, each calibration curve solution), each of the Aβ-related peptide and the internal standard peptide is detected as the signal intensity. That is, signal intensity (A) of the Aβ-related peptide and signal intensity (B) of the internal standard peptide are obtained for each calibration curve solution having a plurality of concentrations of the Aβ-related peptide. The signal intensity is a peak height at a peak corresponding to the Aβ-related peptide or the internal standard peptide in a mass spectrum output as a result of mass spectrometry.

### [Signal standardization step]

In this step, the signal intensity (A) of the Aβ-related peptide is standardized by the signal intensity (B) of the internal standard peptide for each of the plurality of calibration curve solutions.

Specifically, the value of the signal intensity (A) is divided by the value of the signal intensity (B) (A/B). As a result, normalized intensity is obtained for each calibration curve solution. That is, a plurality of normalized intensities having different Aβ-related peptide concentrations are obtained.

If necessary, correction processing or the like for correcting a difference in individual mass spectrometers may be performed on the value (A/B) obtained by dividing the value of the signal intensity (A) by the value of the signal intensity (B). As a result, it is possible to obtain normalized strength linear with respect to the concentration regardless of the difference in individual mass spectrometers.

### [Calculation step]

In this step, a regression equation of a calibration curve is calculated based on the plurality of normalized intensities and the concentration of Aβ-related peptide corresponding thereto.

Specifically, a graph plotting the Aβ-related peptide concentration on the horizontal axis and the normalized intensity on the vertical axis is created, and the regression equation is calculated from this graph to create a regression line.

The type of the regression line may be either a linear regression equation (approximate equation y = ax + b) or a quadratic curve (approximate equation y = ax² + bx + c). Weighting (1/x or 1/x²) may be appropriately set for the regression line.

The calculation of the regression line can be created, for example, by inputting the values of the Aβ-related peptide concentration and the normalized intensity to Excel (registered trademark) and using the graph function of Excel.

In addition to the creation of the regression line, precision and trueness in each plot are calculated, and a suitable use range (usable concentration region) of the regression line can be selected from the precision and the trueness. That is, only a plot having precision of, for example, 25% or less or 20% or less and trueness of within 100 ± 25%, or within 100 ± 20% can be set as a suitable use range.

In the selection of the regression line, at least four types of regression lines of (1) no weighting, weighting 1/x and weighting 1/x² of a linear regression equation and (2) a quadratic curve can be created. Among these, a regression line having the best precision and trueness in a well-balanced manner over a wide density region (x-axis direction range) may be selected, that is, a regression line entirely having a low precision value and trueness close to 100% may be selected.

In the method for creating a calibration curve of the first aspect, an accurate calibration curve can be created. The creation method of the first aspect has been achieved by the following knowledge. In a conventional method for creating a calibration curve, immunoprecipitation and mass spectrometry are sequentially performed using a calibration curve solution obtained by mixing Aβ-related peptide (measurement target) and internal standard peptide (for example, SIL-Aβ related peptide) in BSA-containing PBS to create a calibration curve. However, it has been found that since the Aβ-related peptide (also including SIL-AB-related peptide as an internal standard) has high hydrophobicity, the Aβ-related peptide adheres to a side surface of a test vessel such as a tube, a large loss occurs in each peptide, and the signal intensity of each peptide cannot be correctly detected. Based on this finding, in the method for creating a calibration curve of the first aspect, by using a solution containing a surfactant as a calibration curve solution, adhesion of the peptide to the test vessel is suppressed, that is, loss of the peptide is reduced, so that the signal intensity is more accurately detected, and accuracy of the calibration curve is improved. The precision or the trueness of the resulting calibration curve is also good. The method for creating a calibration curve of the first aspect is not limited to the above knowledge and mechanism.

### 2. Second aspect (method for measuring Aβ-related peptide)

A second aspect is a method for quantifying Aβ-related peptide, in which a concentration of the Aβ-related peptide in a measurement sample is determined using a calibration curve obtained by the creation method. For example, a sample containing the peptide to be measured is provided, immunoprecipitation and mass spectrometry are sequentially performed, and the obtained mass spectrometry result is used to determine the concentration of the peptide to be measured based on the calibration curve. Specifically, the method includes a measurement sample provision step, a first bonding step, a first washing step, a first elution step, a neutralization step, a second bonding step, a second washing step, a second elution step, a detection step, and a concentration determination step.

### [Measurement sample provision step]

In this step, a measurement sample is provided.

The measurement sample contains peptide to be measured (AB-related peptide to be measured in concentration) and internal standard peptide. A known amount of internal standard peptide is mixed with a sample containing a peptide to be measured. As a result, a measurement sample in which the concentration of the peptide to be measured is unknown and the concentration of the internal standard peptide is known is obtained.

Examples of the sample containing the peptide to be measured include a biological sample. Examples of the biological sample include body fluids such as blood, cerebrospinal fluid, urine, body secreting fluid, saliva, and sputum; and examples thereof include feces. The blood includes whole blood, plasma, serum, and the like. The blood may be obtained by subjecting whole blood collected from an individual to treatment such as centrifugation and cryopreservation. In the present analysis method, blood is preferably exemplified. Blood is less invasive than cerebral bone marrow fluid, is a target sample for screening in a medical examination or the like, and is easily available.

The measurement sample may be further mixed with a coupling solution. The coupling solution is, for example, a neutral buffer, and is preferably a neutral buffer containing a surfactant. As the neutral buffer containing a surfactant, those similar to the surfactant and neutral buffer exemplified in the standard solution can be used.

### [First bonding step to detection step]

The first bonding step, the first washing step, the first elution step, the neutralization step, the second bonding step, the second washing step, the second elution step, and the detection step are similar to the respective steps described above in the creation of the calibration curve except that the calibration curve solution is a measurement sample. That is, the same applies except that the measurement sample is brought into contact with the first carrier in the first bonding step. In the detection step, the signal intensity of the peptide to be measured and the signal intensity of the internal standard peptide are obtained as mass spectrometry results.

### [Concentration determination step]

In this step, the peptide to be measured is quantified using the calibration curve with respect to the mass spectrometry result. That is, the signal intensity of the peptide to be measured and the signal intensity of the internal standard peptide are associated with the regression line of the calibration curve to determine a desired concentration of the Aβ-related peptide. For example, the signal intensity of the peptide to be measured is standardized by the signal intensity of the internal standard peptide to calculate normalized intensity. Subsequently, the normalized intensity is fitted to the regression line to determine the concentration.

In this method for measuring Aβ-related peptide, since an accurate calibration curve is used, an accurate Aβ peptide concentration can be measured.

### 3. Third aspect

In the method for creating a calibration curve of the first aspect, two immunoprecipitation methods (affinity purification) are performed in the immunoprecipitation-mass spectrometry step; however, for example, one immunoprecipitation method may be performed. In this case, the immunoprecipitation-mass spectrometry step of the first aspect includes a first bonding step, a first washing step, a second elution step, and a detection step. Each step is the same as in the first aspect. The third aspect also has similar working-effects to the first aspect. From the viewpoint of reliably removing unnecessary components and creating an accurate calibration curve even when an abundance of the Aβ-related peptide or the internal standard peptide is even smaller, the first aspect is preferable.

### 4. Fourth aspect

In the method for measuring Aβ-related peptide of the fourth aspect, two immunoprecipitation methods (affinity purification) are performed at the time of measuring a measurement target; however, for example, one immunoprecipitation method may be performed. In this case, the measurement method of the fourth aspect includes a measurement sample provision step, a first bonding step, a first washing step, a second elution step, a detection step, and a concentration determination step. Each step is the same as in the second aspect. The fourth aspect also has similar working-effects to the second aspect. From the viewpoint of reliably removing unnecessary components and accurately detecting the Aβ-related peptide and the internal standard peptide even when the abundance of the Aβ-related peptide or the internal standard peptide is even smaller, the second aspect is preferable.

### 5. Aspect

It is to be understood by those skilled in the art that the plurality of exemplary embodiments described above are specific examples of the following aspects.
(Clause 1) A method for creating a calibration curve according to one aspect is a method for creating a calibration curve for quantifying amyloid β-related peptide, the method may include the steps of
   providing a plurality of standard solutions containing the amyloid β-related peptide and a surfactant and having different concentrations of the amyloid β-related peptide;
   mixing a solution containing internal standard peptide with each of the plurality of standard solutions to obtain a plurality of calibration curve solutions;
   performing immunoprecipitation and mass spectrometry on the plurality of calibration curve solutions to obtain signal intensity (A) in the amyloid β-related peptide and signal intensity (B) in the internal standard peptide for each of the plurality of calibration curve solutions;
   standardizing the signal intensity (A) with the signal intensity (B) for each of the plurality of calibration curve solutions to obtain a plurality of normalized intensities; and
   calculating a regression equation of a calibration curve based on the plurality of normalized intensities and the concentration of the amyloid β-related peptide corresponding to the plurality of normalized intensities.
(Clause 2) In the creation method according to Clause 1, the surfactant may be a nonionic surfactant.
(Clause 3) In the creation method according to Clause 1 or 2, the amyloid β-related peptide may be at least one selected from the group consisting of Aβ1-40, Aβ1-42, andAPP669-711.
(Clause 4) In the creation method according to any one of Clauses 1 to 3, the immunoprecipitation and the mass spectrometry may include:
   a first bonding step of bringing the calibration curve solution into contact with a first carrier to obtain a first conjugate in which the amyloid β-related peptide is bonded to the first carrier;
   a first washing step of washing the first conjugate;
   a first elution step of bringing the first conjugate into contact with a first acidic solution to obtain a first eluate in which the amyloid β-related peptide is eluted in the first acidic solution;
   a neutralization step of mixing the first eluate with a neutral buffer to obtain a purification solution;
   a second bonding step of bringing the purification solution into contact with a second carrier to obtain a second conjugate in which the amyloid β-related peptide is bonded to the second carrier;
   a second washing step of washing the second conjugate;
   a second elution step of bringing the second conjugate into contact with a second acidic solution to obtain a second eluate in which the amyloid β-related peptide is eluted in the second acidic solution; and
   a detection step of detecting the amyloid β-related peptide in the second eluate by mass spectrometry.
(Clause 5) A method for measuring amyloid β-related peptide according to one embodiment may include quantifying amyloid β-related peptide in a measurement sample using a calibration curve obtained by the creation method according to any one of Clauses 1 to 4.

### EXAMPLES

Next, the present invention will be described in detail with reference to Examples and Comparative Examples; however, the scope of the present invention is not limited thereto.

### Example 1 (creation of calibration curve)

### [Standard solution provision step]

Synthetic peptides of Aβ1-40, Aβ1-42, and APP669-711 (manufactured by Peptide Institute, Inc) as Aβ-related peptides were each dissolved in 0.1% aqueous ammonia to prepare an Aβ1-40 stock solution (1000 pM), an Aβ1-42 stock solution (1000 pM), and an APP669-711 stock solution (200 pM). Each synthetic peptide stock solution was diluted using a surfactant containing buffer (10 mg/mL BSA, 0.1% (w/v) NTM, 0.1% (w/v) DDM, 150 mM NaCl, 50 mM Tris-HCl solution: pH 7.4) as a peptide diluent to prepare a 10 pM Aβ1-40 solution, a 10 pM Aβ1-42 solution, and a 2 pM APP669-711 solution, respectively.

Then, 100 µL of a 10 pM Aβ1-40 solution, 10 µL of a 10 pM Aβ1-42 solution, and 50 µL of a 2 pM APP669-711 solution were mixed with 9840 µL of the peptide diluent (same as above) to prepare a mixed solution (CS 100000) containing Aβ1-40, Aβ1-42, and APP669-711. In addition, a peptide diluent (same as above) was added to CS 100000 to dilute CS 100000 such that Aβ1-40, Aβ1-42 and APP669-711 were at the concentrations given in Table 2. Thus, a standard solution having each concentration was prepared.

The abbreviation of the surfactant is as follows.
DDM: n-Dodecyl-β-D-maltoside
NTM: n-Nonyl-β-D-thiomaltoside

**[Table 2]**

| Name | Concentration (pM) | | |
|---|---|---|---|
| | A*β*1-40 | A*β*1-42 | APP669-711 |
| CS150 | 150 | 15 | 15 |
| CS120 | 120 | 12 | 12 |
| CS100 | 100 | 10 | 10 |
| CS70 | 70 | 7 | 7 |
| CS50 | 50 | 5 | 5 |
| CS30 | 30 | 3 | 3 |
| CS15 | 15 | 1.5 | 1.5 |

### [Calibration curve solution preparation step]

Aβ1-38 (SIL-Aβ1-38) labeled with a stable isotope as internal standard peptide was mixed in a surfactant containing buffer (0.2% (w/v) DDM, 0.2% (w/v) NTM, 800 mM GlcNAc, 100 mM Tris-HCl, 300 mM NaCl: pH 7.4) so as to have 11 pM. Thereby, an internal standard-containing solution was prepared.

To 250 µL of each standard solution (CS15, CS30, CS50, CS70, CS100, CS120, CS150), 250 µL of an internal standard-containing solution was mixed, and then the mixture was allowed to stand on ice for 5 to 60 minutes. As a result, seven types of calibration curve solutions were prepared.

### [Immunoprecipitation-mass spectrometry step]

### (First bonding step, first washing step, first elution step)

Clone 6E10 (Covance Inc.) of an anti-Aβ antibody (IgG) having 3-8 residues of amyloid β protein (AB) as an epitope was provided. Antibody beads were prepared by reacting 24.75 mg of magnetic beads (Dynabeads M-270 Epoxy) with 500 pg of an anti-Aβ antibody (IgG) in an immobilization buffer (0.1 M phosphate buffer containing 1.3 M ammonium sulfate: pH 7.4) at 37°C for 16 to 24 hours.

Each calibration curve solution was mixed with the antibody beads and shaken at 4°C for 1 hour. Thereafter, the antibody beads were washed once with 100 µL of a washing buffer (0.1% (w/v) DDM, 0.1% (w/v) NTM, 50 mM Tris-HCl, 150 mM NaCl: pH 7.4) and washed once with 50 µL of a 50 mM ammonium acetate buffer. Thereafter, the antibody beads were brought into contact with a first acidic solution (50 mM glycine buffer containing 0.1% (w/v) DDM: pH 2.8) to elute the Aβ-related peptide and the internal standard peptide into the first acidic solution. As a result, a first eluate containing the Aβ-related peptide and the internal standard peptide was obtained.

### (Neutralization step)

The first eluate was mixed with a neutral buffer (0.2% (w/v) DDM, 800 mM GlcNAc, 300 mM Tris-HCl, 300 mM NaCl: pH 7.4) to give a purification solution.

### (Second bonding step, second washing step, second elution step)

The purification solution was mixed with the antibody beads and shaken at 4°C for 1 hour. Thereafter, the antibody beads were washed twice with 50 µL of a second washing buffer (0.1% (w/v) DDM, 50 mM Tris-HCl, 150 mM NaCl: pH 7.4), washed once with 50 µL of a 50 mM ammonium acetate buffer, and further washed once with 30 µL of water. Thereafter, the antibody beads were brought into contact with a second eluent (5 mM HCl, 0.1 mM methionine, 70% (v/v) aqueous acetonitrile) to elute the Aβ-related peptide and the internal standard peptide into the second eluate. As a result, the second eluate containing the Aβ-related peptide and the internal standard peptide was obtained.

### (Analysis step)

As a mass spectrometer, a MALDI-TOF MS apparatus (manufactured by Shimadzu Corporation) was used. A0.5 mg/mL CHCA/0.2% (w/v) MDPNAmatrix solution was prepared using α-cyano-4-hydroxycinnamic acid (CHCA) as a matrix for Linear TOF, methylene diphosphonic acid (MDPNA) as a matrix additive, and acetonitrile as a solvent. 0.5 µL each of the matrix solution was added dropwise to 4 wells of a MALDI plate (µFocus MALDI plate 900 µm (Hudson Surface Technology, Inc., Fort Lee, NJ)), and the plate was dried. The second eluate was added dropwise to these 4 wells and placed.

MALDI-TOF MS was then activated to detect the Aβ-related peptide and the internal standard peptide. As setting conditions, mass spectrum data was acquired by Linear TOF in a positive ion mode using AXIMA Performance (Shimadzu/KRATOS, Manchester, UK). The data was accumulated for every 400 spots and every 16,000 shots for 1 well. An m/z value of Linear TOF was expressed as a peak average mass. The m/z value was calibrated using human angiotensin II and human ACTH fragment 18-39 as external standards.

### [Signal standardization step]

In each mass spectrum, the signal intensity of the Aβ-related peptide was normalized by dividing the signal intensity of the Aβ-related peptide by the signal intensity of the internal standard peptide (SIL-Aβ1-38) to obtain normalized intensity. At this time, an average value of the normalized intensity of the mass spectrum of 4 wells was determined. The results are shown in Tables 3 to 5.

### [Calculation step]

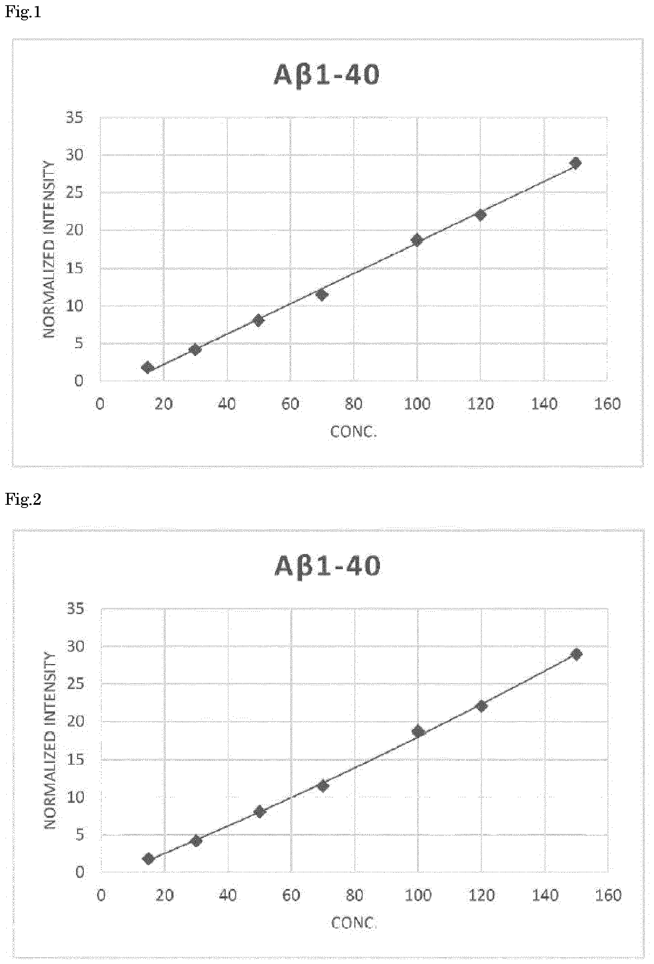

Using Excel (registered trademark), the value of each peptide concentration and the value of the normalized intensity of the standard solution were plotted, and a regression equation was calculated from this plot to create a calibration curve. In the creation of the calibration curve, a linear regression equation by a least squares method or a quadratic curve was used. For the linear regression equation by the least squares method, each of "none", "1/x", and "1/x²" was set as weighting. The results are shown in Tables 3 to 5 and FIGS. 1 to 6.

**[Table 3]**

| Type of regression line | Approximate equation | Item | A*β*1-40 concentration(pM) | | | | | | |
|---|---|---|---|---|---|---|---|---|---|
| | | | 15 | 30 | 50 | 70 | 100 | 120 | 150 |
| Linear regression No weighting (FIG. 1) | Y=0.2021x-1.8430 | Normalized intensity | 1.806 | 4.185 | 8.063 | 11.502 | 18.699 | 22.028 | 28.946 |
| | | Inverse regression value (pM) | 18.1 | 29.8 | 49.0 | 66.0 | 102 | 118 | 152 |
| | | Precision(CV) | 4.4% | 2.5% | 3.9% | 7.3% | 3.0% | 2.5% | 11.3% |
| | | Trueness | 120.4% | 99.4% | 98.0% | 94.3% | 101.6% | 98.4% | 101.6% |
| Linear regression Weighting 1/x | Y=0.1967x-1.4322 | Normalized intensity | 1.806 | 4.185 | 8.063 | 11.502 | 18.699 | 22.028 | 28.946 |
| | | Inverse regression value (pM) | 16.5 | 28.6 | 48.3 | 65.7 | 102 | 119 | 154 |
| | | Precision(CV) | 4.4% | 2.5% | 3.9% | 7.3% | 3.0% | 2.5% | 11.3% |
| | | Trueness | 109.7% | 95.2% | 96.5% | 93.9% | 102.3% | 99.4% | 102.9% |
| Linear regression Weighting 1/x2 | Y=0.1907x-1.1674 | Normalized intensity | 1.806 | 4.185 | 8.063 | 11.502 | 18.699 | 22.028 | 28.946 |
| | | Inverse regression value (pM) | 15.6 | 28.1 | 48.4 | 66.4 | 104 | 122 | 158 |
| | | Precision(CV) | 4.4% | 2.5% | 3.9% | 7.3% | 3.0% | 2.5% | 11.3% |
| | | Trueness | 104.0% | 93.6% | 96.8% | 94.9% | 104.2% | 101.4% | 105.3% |
| Quadratic curve (FIG. 2) | Y=0.0002x²+0.1703x -0.9609 | Normalized intensity | 1.806 | 4.185 | 8.063 | 11.502 | 18.699 | 22.028 | 28.946 |
| | | Inverse regression value (pM) | 160 | 29.2 | 50.1 | 67.9 | 103 | 119 | 150 |
| | | Precision(CV) | 4.4% | 2.5% | 3.9% | 7.3% | 3.0% | 2.5% | 11.3% |
| | | Trueness | 106.4% | 97.4% | 100.2% | 97.0% | 103.2% | 99.0% | 99.9% |

**[Table 4]**

| Type of regression line | Approximate equation | Item | A*β*1-42 concentration (pM) | | | | | | |
|---|---|---|---|---|---|---|---|---|---|
| | | | 1.5 | 3 | 5 | 7 | 10 | 12 | 15 |
| Linear regression No weighting (FIG. 3) | Y=0.0615x-0.0505 | Normalized intensity | 0.067 | 0.140 | 0.244 | 0.351 | 0.563 | 0.670 | 0.900 |
| | | Inverse regression value (pM) | 1.92 | 3.10 | 4.79 | 6.53 | 100 | 11.7 | 15.5 |
| | | Precision(CV) | 6.5% | 6.7% | 7.1% | 7.1% | 2.8% | 3.0% | 8.0% |
| | | Trueness | 127.7% | 103.3% | 95.7% | 93.3% | 99.8% | 97.7% | 103.1% |
| Linear regression Weighting 1/x | Y=0.0590x-0.0317 | Normalized intensity | 0.067 | 0.140 | 0.244 | 0.351 | 0.563 | 0.670 | 0.900 |
| | | Inverse regression value (pM) | 1.68 | 2.91 | 4.67 | 6.49 | 10.1 | 11.9 | 15.8 |
| | | Precision(CV) | 6.5% | 6.7% | 7.1% | 7.1% | 2.8% | 3.0% | 8.0% |
| | | Trueness | 111.8% | 97.0% | 93.3% | 92.7% | 100.8% | 99.1% | 105.2% |
| Linear regression Weighting 1/x2 | Y=0.0566x-0.0213 | Normalized intensity | 0.067 | 0.140 | 0.244 | 0.351 | 0.563 | 0.670 | 0.900 |
| | | Inverse regression value (pM) | 1.56 | 2.85 | 4.68 | 6.58 | 10.3 | 12.2 | 16.3 |
| | | Precision(CV) | 6.5% | 6.7% | 7.1% | 7.1% | 2.8% | 3.0% | 8.0% |
| | | Trueness | 104.2% | 95.0% | 93.6% | 93.9% | 103.1% | 101.8% | 108.4% |
| Quadratic curve (FIG. 4) | Y=0.0010x²+0.0444x -0.0031 | Normalized intensity | 0.067 | 0.140 | 0.244 | 0.351 | 0.563 | 0.670 | 0.900 |
| | | Inverse regression value (pM) | 1.53 | 3.01 | 4.97 | 6.86 | 10.3 | 11.8 | 150 |
| | | Precision(CV) | 6.5% | 6.7% | 7.1% | 7.1% | 2.8% | 3.0% | 8.0% |
| | | Trueness | 102.0% | 100.3% | 99.5% | 98.1% | 102.6% | 98.7% | 100.1% |

**[Table 5]**

| Type of regression line | Approximate equation | Item | APP669-711 concentration(pM) | | | | | | |
|---|---|---|---|---|---|---|---|---|---|
| | | | 1.5 | 3 | 5 | 7 | 10 | 12 | 15 |
| Linear regression No weighting (FIG.5) | Y=0.0649x-0.0653 | Normalized intensity | 0.065 | 0.136 | 0.242 | 0.348 | 0.591 | 0.695 | 0.938 |
| | | Inverse regression value (pM) | 2.01 | 3.10 | 4.74 | 6.37 | 10.1 | 11.7 | 15.5 |
| | | Precision (CV) | 6.8% | 4.0% | 2.8% | 2.8% | 2.7% | 1.0% | 9.3% |
| | | Trueness | 133.9% | 103.2% | 94.7% | 90.9% | 101.1% | 97.7% | 103.1% |
| Linear regression Weighting 1/x | Y=0.0618x-0.0418 | Normalized intensity | 0.065 | 0.136 | 0.242 | 0.348 | 0.591 | 0.695 | 0.938 |
| | | Inverse regression value (pM) | 1.73 | 2.87 | 4.59 | 6.30 | 10.2 | 11.9 | 15.9 |
| | | Precision (CV) | 6.8% | 4.0% | 2.8% | 2.8% | 2.7% | 1.0% | 9.3% |
| | | Trueness | 115.1% | 95.6% | 91.8% | 90.0% | 102.3% | 99.4% | 105.7% |
| Linear regression Weighting 1/x2 | Y=0.0587x-0.0280 | Normalized intensity | 0.065 | 0.136 | 0.242 | 0.348 | 0.591 | 0.695 | 0.938 |
| | | Inverse regression value (pM) | 1.58 | 2.79 | 4.60 | 6.40 | 10.5 | 12.3 | 16.5 |
| | | Precision (CV) | 6.8% | 4.0% | 2.8% | 2.8% | 2.7% | 1.0% | 9.3% |
| | | Trueness | 105.6% | 92.9% | 92.0% | 91.5% | 105.5% | 102.7% | 109.8% |
| Quadratic curve (FIG . 6) | Y=0.0011x²+0.0450x -0.0101 | Normalized intensity | 0.065 | 0.136 | 0.242 | 0.348 | 0.591 | 0.695 | 0.938 |
| | | Inverse regression value (pM) | 1.60 | 2.99 | 4.94 | 6.72 | 10.4 | 11.9 | 15.0 |
| | | Precision (CV) | 6.8% | 4.0% | 2.8% | 2.8% | 2.7% | 1.0% | 9.3% |
| | | Trueness | 106.6% | 99.8% | 98.8% | 96.0% | 104.1% | 98.8% | 99.8% |

### 2. Evaluation of calibration curve

For each regression line, an inverse regression value, precision, and trueness were obtained. For the trueness, within 100 ± 25% at a minimum concentration (15 pM for Aβ1-40, 1.5 pM for Aβ1-42 and APP669-711) was considered to be appropriate, and within 100 ± 20% at the other concentrations was considered to be appropriate. For the precision (CV), 25% or less at a minimum concentration was considered to be appropriate, and 20% or less at the other concentrations was considered to be appropriate. Among seven points, at least six consecutive points were considered to be fitted in the above allowable range. The fitted range was defined as an effective range of an absolute quantification method.

Aβ1-40: As can be seen from Table 3, in no weighting (FIG. 1), 1/x and 1/x² of the linear regression equation and the regression equation of the quadratic curve (FIG. 2), the precision and the trueness of all seven points in a range of 15 to 150 pM were appropriate.

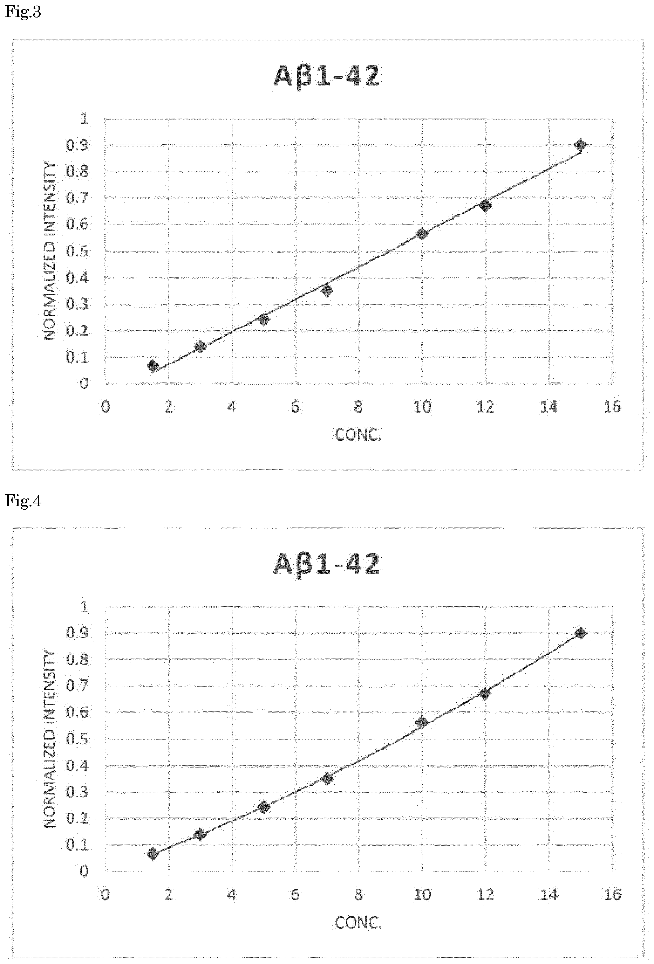

Aβ1-42: As can be seen from Table 4, in 1/x and 1/x² of the linear regression equation, and the regression equation of the quadratic curve (FIG. 4), the precision and the trueness of all seven points in a range of 1.5 to 15 pM were appropriate. On the other hand, in the case of no weighting of the linear regression equation (FIG. 3), since the trueness was 125% or more only for 1.5 pM, the precision and the trueness of six points in a range of 3 to 15 pM were appropriate.

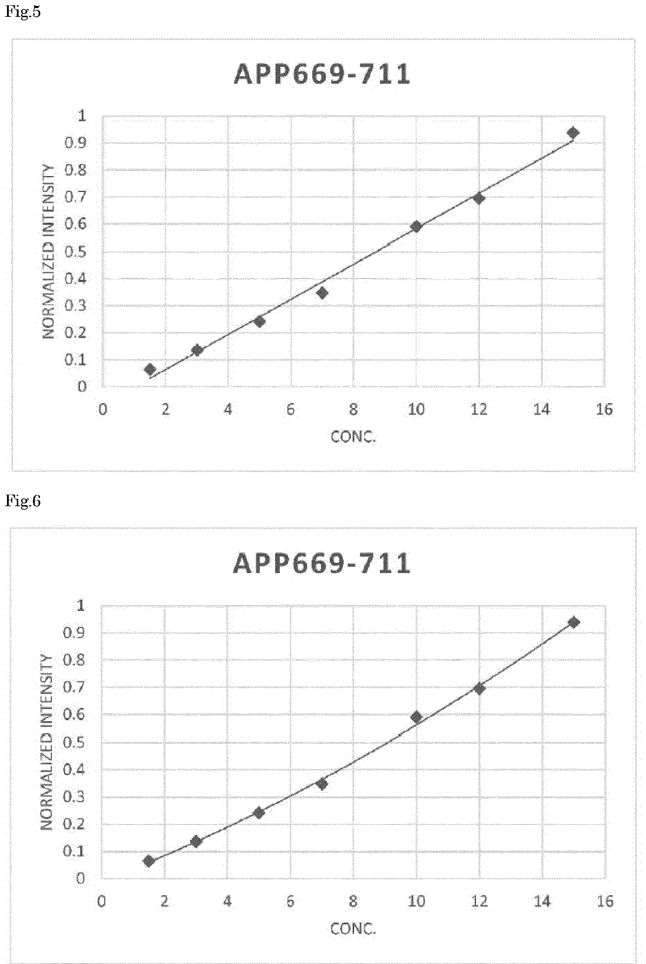

APP669-711: As can be seen from Table 5, in 1/x and 1/x² of the linear regression equation, and the regression equation of the quadratic curve (FIG. 6), the precision and the trueness of all seven points in a range of 1.5 to 15 pM were appropriate. On the other hand, in the case of no weighting of the linear regression equation (FIG. 5), since the trueness was 125% or more only for 1.5 pM, the precision and the trueness of six points in the range of 3 to 15 pM were appropriate.

It was found that Aβ1-40, Aβ1-42, and APP669-711 could calculate the concentration of the measurement sample using the regression equation of the calibration curve in these appropriate ranges.

### 3. Peptide adsorption experiment

### [Preparation Example 1]

As internal standard peptide, SIL-Aβ1-38 was mixed with a buffer (1 mg/mL BSA, 150 mM NaCl, 40 mM Tris-HCl) so as to have a concentration of 1 pM. Operation of transferring the peptide solution to a new 0.5 mL tube was performed twice.

Then, the peptide solution was diluted with a buffer (0.2% (w/v) DDM, 0.2% (w/v) NTM, 800 mM GlcNAc, 100 mM Tris-HCl, 300 mM NaCl: pH 7.4) so that the peptide concentration was 1/100 (that is, the concentration equivalent to 10 pM). 250 µL of this diluent was mixed with 250 µm of human plasma (Lot: R294375) and then allowed to stand on ice for 5 to 60 minutes.

A method similar to that in the above [Immunoprecipitation-mass spectrometry] was performed except that the obtained human plasma mixture was used instead of the calibration curve solution. As a result, the signal intensity of SIL-Aβ1-38 and the signal intensity of Aβ1-40 contained in human plasma were obtained.

### [Preparation Example 2]

DDM was added to a buffer (1 mg/mL BSA, 150 mM NaCl, 40 mM Tris-HCl) to a concentration of 0.1% (w/v) to prepare a surfactant containing buffer. Production was performed in the same manner as in Preparation Example 1 except that this surfactant containing buffer was used.

### [Preparation Example 3]

Tween-20 (polyoxyethylene sorbitan monolaurate) was added to a buffer (1 mg/mL BSA, 150 mM NaCl, 40 mM Tris-HCl) to a concentration of 0.05% (w/v) to prepare a surfactant containing buffer. Production was performed in the same manner as in Preparation Example 1 except that this surfactant containing buffer was used.

### [Results of evaluation of adsorption of peptide]

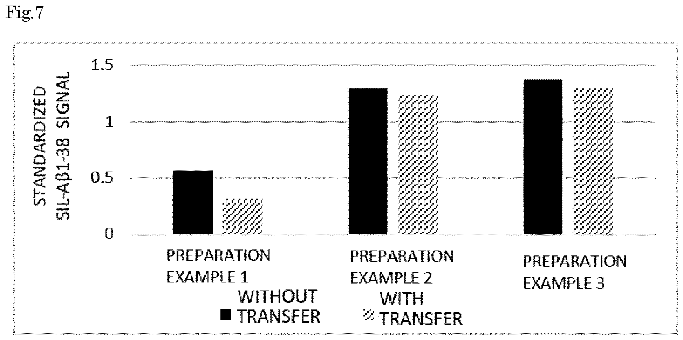

In this adsorption experiment, human plasma of the same Lot (R294375) were used in all conditions, and the concentration of Aβ1-40 contained in the human plasma was the same. Thus, in each preparation example, the signal intensity of SIL-Aβ1-38 was divided by the signal intensity of Aβ1-40 to calculate a value obtained by standardizing the signal intensity of SIL-Aβ1-38. These values are shown in FIG. 7.

In each preparation example, the same preparation was performed when the operation of transferring the peptide solution to a new tube was not carried out. The standardized values at this time are also shown in FIG. 7.

As can be seen from FIG. 7, in Preparation Example 1 (without surfactant), the value with the transfer operation was reduced to 55% of the value without the transfer operation. On the other hand, in Preparation Example 2 (containing DDM) and Preparation Example 3 (containing Tween-20), the value with the transfer operation was 94 to 95% of the value without the transfer operation, and there was almost no change in both values. Regarding a standardized SIL-Aβ1-38 signal without the transfer operation, Preparation Examples 2 and 3 showed higher values than Preparation Example 1. From this result, it was confirmed that Preparation Examples 2 and 3 could suppress a decrease in signal intensity of the peptide (standardized SIL-Aβ1-38), that is, measurement loss of the peptide. It is presumed that the decrease in signal in Preparation Example 1 is caused by adsorption of the peptide to a tube wall surface due to hydrophobic interaction, and the adsorption can be suppressed by the inclusion of the surfactant.

## Claims

1. A method for creating a calibration curve for quantifying amyloid β-related peptide, the method comprising the steps of
providing a plurality of standard solutions containing the amyloid β-related peptide and a surfactant and having different concentrations of the amyloid β-related peptide;
mixing a solution containing internal standard peptide with each of the plurality of standard solutions to obtain a plurality of calibration curve solutions;
performing immunoprecipitation and mass spectrometry on the plurality of calibration curve solutions to obtain signal intensity (A) in the amyloid β-related peptide and signal intensity (B) in the internal standard peptide for each of the plurality of calibration curve solutions;
standardizing the signal intensity (A) with the signal intensity (B) for each of the plurality of calibration curve solutions to obtain a plurality of normalized intensities; and
calculating a regression equation of a calibration curve based on the plurality of normalized intensities and the concentration of the amyloid β-related peptide corresponding to the plurality of normalized intensities.

2. The creation method according to claim 1, wherein the surfactant is a nonionic surfactant.

3. The creation method according to claim 1, wherein the amyloid β-related peptide is at least one selected from the group consisting of Aβ1-40, Aβ1-42, andAPP669-711.

4. The creation method according to claim 1, wherein the immunoprecipitation and the mass spectrometry includes:
a first bonding step of bringing the calibration curve solution into contact with a first carrier to obtain a first conjugate in which the amyloid β-related peptide is bonded to the first carrier;
a first washing step of washing the first conjugate;
a first elution step of bringing the first conjugate into contact with a first acidic solution to obtain a first eluate in which the amyloid β-related peptide is eluted in the first acidic solution;
a neutralization step of mixing the first eluate with a neutral buffer to obtain a purification solution;
a second bonding step of bringing the purification solution into contact with a second carrier to obtain a second conjugate in which the amyloid β-related peptide is bonded to the second carrier;
a second washing step of washing the second conjugate;
a second elution step of bringing the second conjugate into contact with a second acidic solution to obtain a second eluate in which the amyloid β-related peptide is eluted in the second acidic solution; and
a detection step of detecting the amyloid β-related peptide in the second eluate by mass spectrometry.

5. A method for measuring amyloid β-related peptide comprising quantifying amyloid β-related peptide in a measurement sample using a calibration curve obtained by the creation method according to claim 1.
